# FASCICULE DE BREVET EUROPEEN

(11) **EP 2 837 907 B1**
(45) Date de publication et mention de la délivrance du brevet: **23.08.2017**
(21) Numéro de dépôt: 14306267.7
(22) Date de dépôt: 12.08.2014
(51) Int. Cl.: G01B 11/245, G01B 21/04, A61B 5/107, G06T 7/00, H04N 13/00

(54) **Procédé et installation pour l'acquisition automatique de surfaces tridimensionnelles**
Verfahren und Anlage zur automatischen Erfassung von dreidimensionalen Flächen
Method and device for automatic acquisition of three-dimensional surfaces

(30) Priorité: 14.08.2013 FR 1358004
(43) Date de publication de la demande: 18.02.2015
(73) Titulaire: Telmat Industrie (Société Anonyme), 68360 Soultz (FR)
(72) Inventeur: Dudkiewicz, Gilbert, 68270 Ruelisheim (FR); Keiter, Pascal, 68740 Hirtzfelden (FR); Frey, Frédéric, 68110 Illzach (FR)
(74) Mandataire: Nuss, Laurent

(56) Documents cités:
- EP-A2- 1 091 186
- CH-A2- 703 385
- FR-A1- 2 921 478
- RIZWAN MACKNOJIA ET AL: "Calibration of a network of Kinect sensors for robotic inspection over a large workspace", ROBOT VISION (WORV), 2013 IEEE WORKSHOP ON, IEEE, 15 janvier 2013 (2013-01-15), pages 184-190, XP032415741, DOI: 10.1109/WORV.2013.6521936 ISBN: 978-1-4673-5646-6
- Anonymous: "Multi-Kinect camera calibration | Doc-Ok.org", Doc-ok.org blog, 24 janvier 2013 (2013-01-24), XP055091812, Extrait de l'Internet: URL:http://doc-ok.org/?p=295 [extrait le 2013-12-05]
- JING TONG ET AL: "Scanning 3D Full Human Bodies Using Kinects", IEEE TRANSACTIONS ON VISUALIZATION AND COMPUTER GRAPHICS, IEEE SERVICE CENTER, LOS ALAMITOS, CA, US, vol. 18, no. 4, 4 avril 2012 (2012-04-04), pages 643-650, XP011490419, ISSN: 1077-2626, DOI: 10.1109/TVCG.2012.56

## Description

La présente invention concerne le domaine de l'acquisition de représentations tridimensionnelles d'objets ou de scènes situé(e)s dans un espace d'acquisition, en particulier dans un contexte d'application à l'acquisition d'enveloppes superficielles de sujets humains, par exemple dans un but de mensuration, de modélisation et/ou de classification, en particulier en relation avec l'industrie et le commerce de l'habillement.

Plus précisément, l'invention concerne un procédé et une installation d'acquisition automatique, de traitement et de restitution numérique de surfaces tridimensionnelles.

De nombreux procédés et systèmes aptes à réaliser l'acquisition de surfaces tridimensionnelles et la reconstruction postérieure d'une surface unique et d'un seul tenant sont déjà connus dans l'état de la technique, mettant en oeuvre des moyens différents et/ou des méthodes spécifiques, mais présentant tous des inconvénients ou au moins des limitations notables.

Ainsi, on connaît, par exemple par les documents EP-A-1 207 367, FR-A-2 963 673 et WO 2009/153446, des systèmes dans lesquels des motifs de lumière structurés sont projetés dans l'espace d'acquisition et l'image tridimensionnelle est reconstruite à partir d'au moins deux prises de vue. En plus de nécessiter des moyens de projection spécifiques, le cas échéant du type laser, ce type de système est plus ou moins sensible à la lumière ambiante et la précision de la reconstruction de l'image tridimensionnelle peut, du fait de la complexité de la fusion de surfaces à réaliser, ne pas être suffisante lorsque des mesures fines sont à effectuer.

Pour éviter l'opération complexe de fusionnement des images de différents capteurs, il peut être prévu de n'utiliser qu'un seul capteur, en prévoyant le déplacement contrôlé de ce dernier et/ou du sujet. Néanmoins, cette solution ne fournit généralement pas d'image finale de bonne qualité (résolution insuffisante) et en outre l'acquisition totale prend un temps trop long (possible mouvement du sujet pendant l'acquisition).

Par ailleurs, de nouveaux capteurs 3D de profondeur sont récemment apparus sur le marché grand public (donc à des coûts faibles), par exemple ceux du type connu sous la désignation Carmine® par la société Primesense (et notamment mis en oeuvre dans la console de jeux vidéo Kinect®).

Ces capteurs se présentent chacun sous la forme d'une combinaison de plusieurs dispositifs, l'un qui projette un motif ou mouchetis de points dans le spectre non visible (infrarouge), un autre qui fournit pour chaque pixel acquis également une information de profondeur, et un troisième qui fournit la texture (couleur) du pixel. Les informations collectées en sortie du capteur consistent ainsi en des coordonnées 3D (x, y, z), indiquées par rapport à un référentiel spatial centré sur le centre optique du capteur.

Ce type de capteur délivre les informations 3D à une cadence jusqu'à 60 images/seconde, présente un encombrement réduit et peut être directement raccordé à une unité informatique (par exemple au niveau d'un port USB).

Toutefois, le champ d'acquisition, et notamment la partie de ce champ qui fournit une précision d'acquisition suffisante pour les applications mentionnées en introduction, est limité, tant du point de vue de la profondeur (de 0,35 à 1,4 m environ) que du point de vue de l'ouverture angulaire (54° en horizontal et 45° en vertical).

Il a déjà été proposé de mettre en oeuvre ce type de capteur dans le domaine de l'acquisition tridimensionnelle et dans des systèmes de prise de vue.

Dans un premier type de réalisation connu ("KINECT AVATAR: FULLY AUTOMATIC BODY CAPTURE USING A SINGLE KINECT", Didier Striker et al., DFKI, Kaiserslautern), un seul capteur était utilisé, avec donc les inconvénients précités.

Dans une seconde réalisation connue, plusieurs capteurs étaient montés sur une structure support en définissant un espace d'acquisition, chaque capteur fournissant une image de bonne résolution d'une surface limitée donnée. Toutefois, ce système nécessite la mise en place d'un objet d'étalonnage pour l'opérateur pour calibrer le système (pas de fonctionnement entièrement automatique) et l'assemblage des différentes portions de surface est réalisé par recalage de leurs bords périphériques, nécessitant des traitements complexes et un repositionnement, difficile à réaliser en temps réel, sauf à disposer de ressources de calcul très importantes, et donc trop coûteuses pour les applications visées.

Enfin, par la publication "Calibration of a network of Kinect sensors for robotic inspection over a large workspace", Rizwan Macknojia et al., Robot Vision, 2013, IEEE, 15 janvier 2013, pages 184 à 190, on connaît déjà un procédé correspondant sensiblement au préambule de la revendication 1 et une installation correspondant sensiblement au préambule de la revendication 5.

Le procédé et l'installation divulgués par cette publication permettent de surmonter au moins les principales limitations précitées.

En partant de cet état de la technique, l'invention a pour but de proposer un procédé et une installation améliorés autorisant une automatisation aisée, rapide et précise de la phase d'étalonnage et de calibration.

Ce but est atteint par les caractéristiques des parties caractérisantes des revendications 1 et 5.

L'invention sera mieux comprise grâce à la description ci-après, qui se rapporte à un mode de réalisation préféré, donné à titre d'exemple non limitatif, et expliqué avec référence aux dessins schématiques annexés, dans lesquels :
les figures 1A et 1B sont des vues schématiques partielles en perspective d'une installation selon deux variantes de réalisation de l'invention, seuls les repères visuels et les capteurs étant représentés ;
les figures 2A et 2B sont des vues de dessus d'un étage de l'espace d'acquisition (défini par quatre montants équipés de repères visuels) et des capteurs (quatre) situés à cet étage de l'installation de la figure 2A, ces deux figures indiquant les capteurs actifs respectivement durant une première séquence d'étalonnage (2A) et durant une seconde séquence d'étalonnage (2B) ;
la figure 3 est une vue en coupe et de dessus similaire aux figures 2A et 2B d'une variante de réalisation (montants à section triangulaire), seul un capteur étant représenté ;
les figures 4A et 4B sont des représentations schématiques de dessus d'un étage de l'espace d'acquisition et des capteurs selon deux variantes de réalisation de l'installation (à six capteurs par étage), la figure 4B concernant l'installation de la figure 1B, et
les figures 5A et 5B illustrent des parties d'enveloppes tridimensionnelles obtenues par assemblage de plusieurs (deux ou trois) représentations de surfaces tridimensionnelles avec fusion des zones périphériques chevauchantes.

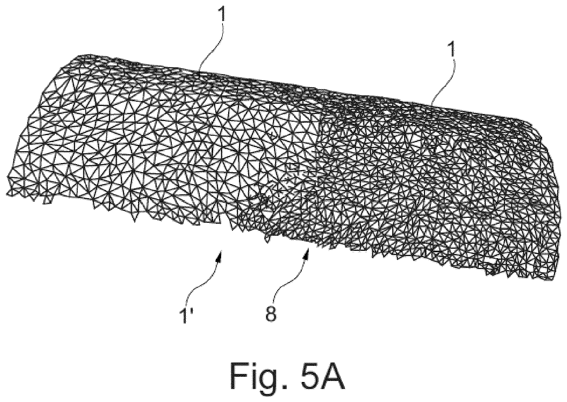

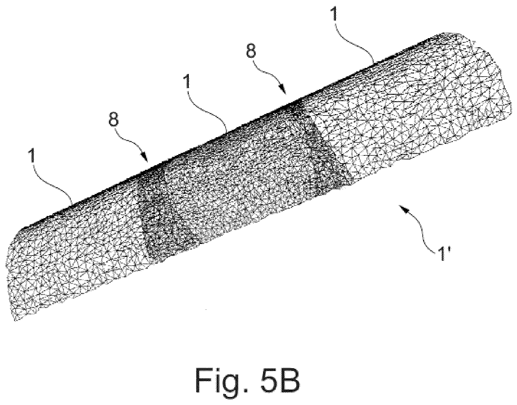

L'invention a pour objet un procédé d'acquisition automatique, de traitement et de restitution numérique de plusieurs surfaces tridimensionnelles 1 délimitant par coopération une enveloppe d'un corps physique ou une scène, en particulier l'enveloppe l' d'un corps humain entier (seule une portion minime est représentée sur les figures 5A et 5B).

Le sujet dont l'enveloppe est à relever est placé ou se place dans un espace d'acquisition 2 situé dans une structure support 3 pour des moyens capteurs 4 et des éléments de repères visuels 5 faisant partie d'une installation 6 d'acquisition, de traitement, de restitution et de stockage de représentations d'enveloppes ou de scènes tridimensionnelles 1'.

La structure support 3 (dont seuls les montants 10 et les potences des colonnes 11, 11' sont représentés) fait généralement partie d'une cabine ou analogue (non représentée).

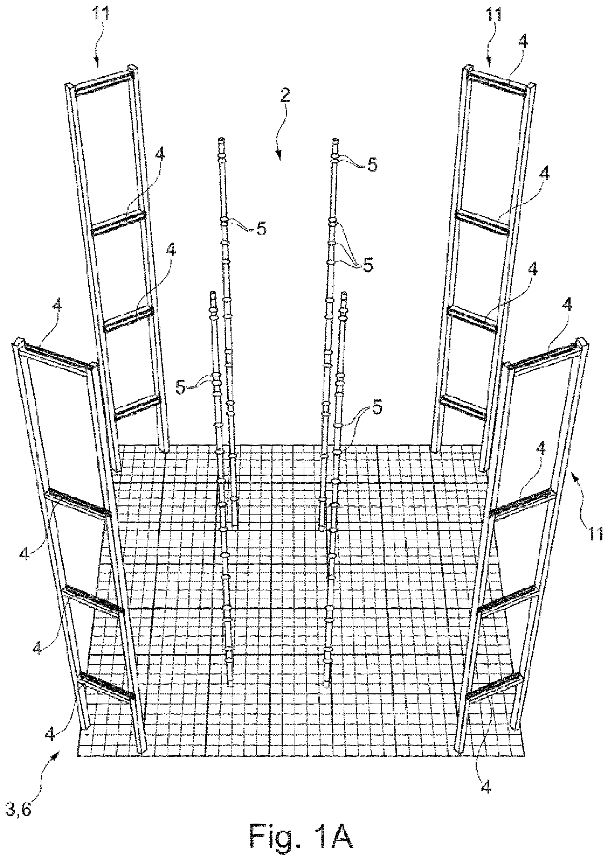

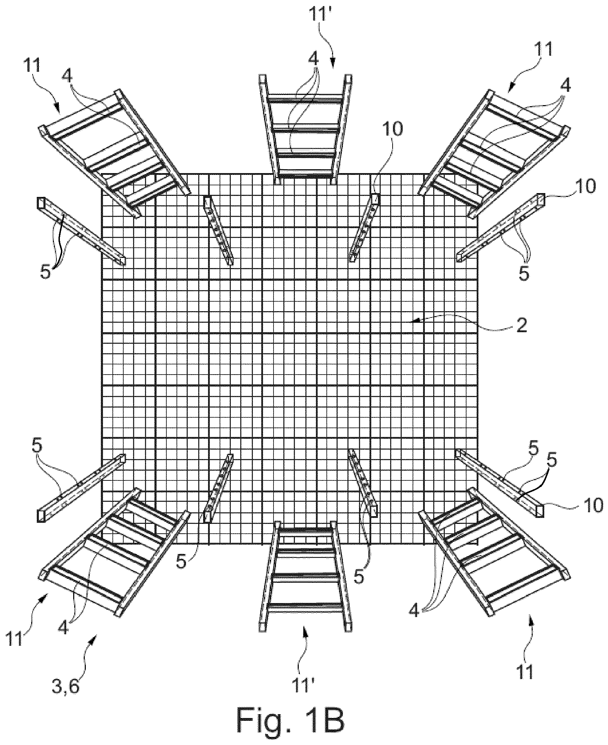

Comme le montrent les figures 1 à 4, cette installation 6 comprend essentiellement, d'une part, une pluralité de capteurs 4 situés en dehors de l'espace d'acquisition 2 et arrangés pour couvrir ensemble la totalité de cet espace avec recouvrement partiel périphérique de leurs champs d'acquisition individuels 7, et, d'autre part, une pluralité de repères visuels 5 actifs positionnés aux limites de l'espace d'acquisition 2, de manière répartie, et parmi lesquels au moins quatre repères non disposés dans un même plan sont situés dans le champ d'acquisition de chacun des capteurs, chaque capteur 4 projetant (ou étant apte à projeter) un motif de points dans le spectre non visible et étant apte à fournir les coordonnées (x, y, z) dans son repère ou référentiel spatial de chaque point d'une surface tridimensionnelle 1 visible dans son champ d'acquisition 7.

Le procédé consiste à réaliser une phase d'étalonnage et de calibration de l'installation 6, pendant laquelle des matrices de transformation ou de conversion de coordonnées entre chacun des repères ou référentiels spécifiques aux différents capteurs 4 et un repère ou un référentiel spatial unique associé à l'installation 6 sont calculées, ce sur la base de l'acquisition des coordonnées d'au moins quatre repères visuels 5 non coplanaires par chaque capteur 4, et, pour chaque nouvelle acquisition de représentation de surfaces tridimensionnelles 1 par les différents capteurs 4, à convertir les coordonnées des voxels desdites représentations numériques partielles dans le référentiel spatial associée à l'installation 6 et à constituer la représentation tridimensionnelle 1' complète de l'enveloppe ou de la scène par assemblage ordonné des différentes représentations partielles avec fusion naturelle (sans traitement mathématique) desdites surfaces au niveau de leurs zones périphériques mutuellement superposées ou chevauchantes 8.

Bien entendu, le positionnement précis de chacun des repères visuels 5 dans le référentiel spatial commun lié à l'installation est connu avec une grande précision.

Conformément à l'invention, la phase d'étalonnage et de calibration est réalisée automatiquement, sur la base de l'acquisition des coordonnées d'au moins quatre repères visuels 5 actifs non coplanaires par chaque capteur 4, et en ce qu'il consiste, durant cette phase, à identifier les repères visuels actifs 5, sous forme de points lumineux, par un codage temporel, en appliquant une séance d'allumage et d'extinction déterminée unique pour chaque repère 5 et à moduler individuellement en intensité chaque repère visuel 5, sous forme de point lumineux, jusqu'à ce que l'image de ce repère 5 acquise par le capteur 4 concerné corresponde à un unique pixel.

Ainsi, l'invention fournit non seulement les moyens pour automatiser aisément la phase d'étalonnage et de calibration, mais également pour augmenter sa précision et accélérer son exécution.

A l'issue de l'étalonnage, le procédé selon l'invention permet d'acquérir en temps réel, avec une pluralité de capteurs 4 et avec une très bonne résolution, une pluralité de surfaces tridimensionnelles 1, dont les représentations numériques peuvent, du fait de l'expression des coordonnées de l'ensemble des points dans un même référentiel spatial, être fusionnées naturellement pour constituer la représentation globale 1' de l'enveloppe du sujet.

L'assemblage avec fusion périphérique des représentations des surfaces tridimensionnelles 1 ne nécessite aucun recalage mathématique, donc aucune manipulation de données susceptible de générer des erreurs.

Les différentes matrices de conversion et de transformation linéaire déterminées lors de la phase préalable de calibration, peuvent notamment être calculées par une méthode des moindres carrés, ce afin de minimiser globalement les erreurs. au niveau des différents facteurs de conversion.

Le procédé selon l'invention, du fait notamment des capteurs 3D 4 utilisés (par exemple du type de ceux indiqués précédemment), ne nécessite aucune projection de lumière supplémentaire et n'est pas sensible à la lumière ambiante (les capteurs 4 projetant un motif de points infrarouge répartis dans le champ d'acquisition 7).

Dans une configuration idéale, il serait souhaitable de disposer d'une matrice de repères 5 répartis de matière homogène sur l'ensemble de la scène observée par chaque capteur 4, ce qui permettrait de corriger de manière optimale les éventuelles aberrations géométriques desdits capteurs 4.

Toutefois, une telle construction optimale est difficile à réaliser dans la pratique compte tenu du nombre extrêmement élevé de repères 5 qu'il y aurait lieu de mettre en place et de la complexité structurelle qui en résulterait.

A titre de compromis avantageux entre une bonne qualité d'acquisition et un nombre limité de repères 5, l'invention peut consister à positionner spatialement les repères visuels 5 par rapport aux différents capteurs 4 de telle manière que parmi les au moins quatre repères 5 non coplanaires vus par chaque capteur 4, au moins un est situé dans la région centrale du champ d'acquisition 7 du capteur 4 concerné et au moins un, préférentiellement plusieurs, dans une zone périphérique dudit champ 7.

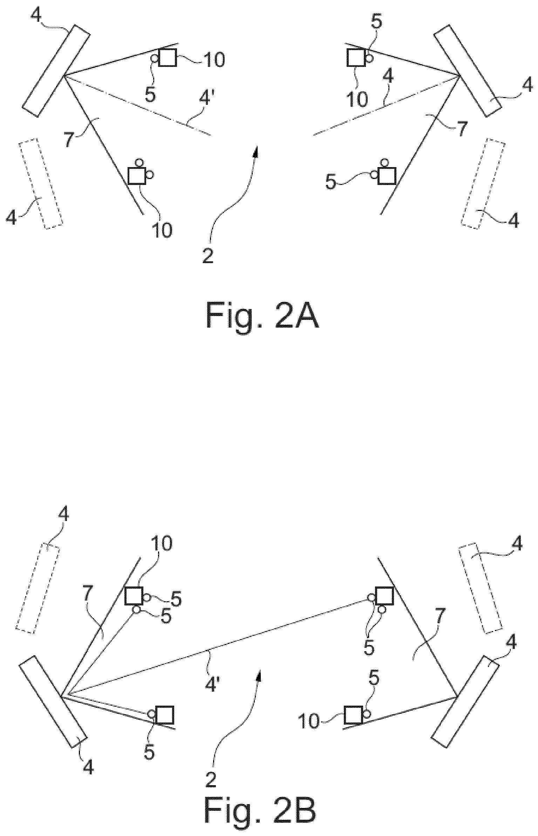

La figure 2B illustre, à titre d'exemple, l'acquisition des coordonnées de quatre repères 5 par un capteur 4, trois des repères étant situés dans un même plan, par exemple le plan du capteur, et le quatrième repère étant situé dans un plan supérieur ou inférieur (ce repère non coplanaire étant confondu sur la figure 2B avec le repère superposé situé dans le même plan que les deux autres repères).

En vue d'aboutir à une bonne qualité de la fusion des surfaces 1 voisines entre elles et donc à une absence de discontinuité dans l'enveloppe, l'invention prévoit avantageusement des zones de recouvrement ou de chevauchement mutuel représentant au moins 10 % entre champs d'acquisition 7 de capteurs 4 voisins.

En outre, selon une caractéristique préférée de l'invention, deux capteurs 4 voisins, dont les champs d'acquisition 7 se chevauchent partiellement en périphérie, partagent au moins un repère visuel commun 5 situé dans ladite région de chevauchement mutuel, lesdits repères visuels actifs 5 se présentant préférentiellement chacun sous la forme d'une diode électroluminescente, éventuellement disposée derrière une surface perforée et dont l'axe d'émission est avantageusement disposé perpendiculairement à l'axe optique du ou des capteurs 4 dont le(s) champ(s) d'acquisition 7 l'englobe(nt).

Enfin, le procédé selon l'invention peut également consister à réaliser virtuellement au moins une projection planaire des points de la représentation numérique tridimensionnelle globale 1' sur au moins un plan, à en extraire une ou plusieurs ligne(s) de contour et à effectuer des mesures et/ou des localisations de zones ou de points remarquables, sur cette ou ces lignes.

A titre d'exemple de réalisation pratique, le processus d'étalonnage peut comprendre les étapes suivantes :
- acquisition d'une séquence de n images,
- localisation des différents points lumineux (repères 5) dans chaque image prise par chaque capteur 4,
- relevé de la séquence d'allumage et d'extinction correspondant à chaque point lumineux pour l'identifier et récupérer sa position géométrique dans le repère du capteur 4 considéré (coordonnées x, y, z),
- calcul de la matrice de transfert pour convertir les coordonnées indiquées dans le repère des capteurs 4 dans le repère de l'installation 6.

De même, à titre d'exemple de réalisation pratique du processus d'acquisition, il peut être prévu de réaliser :
- pour chaque capteur : une acquisition d'une séquence de n images et le moyennage de ces images pour filtrer le bruit,
- l'application de la matrice de transfert pour convertir les coordonnées des points des surfaces 1 exprimées dans le repère du capteur concerné en des coordonnées exprimées dans le repère de l'installation 6.

Enfin, le processus de reconstruction surfacique peut, par exemple, consister à rassembler les points des représentations des surfaces fournis par tous les capteurs et dont les coordonnées sont exprimées dans le repère de l'installation et à réaliser la fusion des surfaces au niveau des zones en chevauchement (figures 5A et 5B).

Le processus de fusion des surfaces peut, par exemple, être similaire à celui mis en oeuvre dans le système Symcad II commercialisé par la demanderesse.

A titre d'exemple pratique non limitatif, et aux fins d'exploitation, les nuages de points (images des points projetés) provenant des différents capteurs 4 sont collectés et peuvent être injectés dans un dispositif (logiciel) de maillage de points (au niveau de l'unité 9) afin de procéder à une simplification en vue de pouvoir construire un modèle géométrique pour en faire une représentation graphique. Il existe des logiciels de maillage ("mailleurs") comme par exemple "Meshlab" (logiciel de traitement de maillages 3D avec de nombreuses fonctionnalités de manipulation d'images 3D).

Toujours à titre d'exemple et en vue d'obtenir une représentation graphique sur l'écran d'un ordinateur de l'ensemble des données acquises et propre et aussi afin de rendre fluide sa manipulation (rotation, translation, zoom, ...), un certain nombre de traitements connus de l'homme du métier sont appliqués aux surfaces 1 :
- Décimation des points 3D afin d'alléger les nuages de points ;
- Lissage des nuages de points 3D afin d'obtenir un objet plus lisse (élimination d'éventuels points parasites), ce qui donne un meilleur aspect à la surface 1, chaque point 3D étant remplacé par la moyenne avec son voisinage (lissage par la méthode du Laplacien) ;
- Création de l'objet volumique par une reconstruction sous forme de triangle (triangulation de Delaunay, méthode de Poisson, ...), avec fusion naturelle au niveau des zones 8 adjacentes/chevauchantes ;
- Application d'un rendu afin de colorer l'objet ou de lui donner l'aspect réel en appliquant la véritable texture de l'objet acquise par chaque capteur 4 lors de l'acquisition ;
- Export des données dans un format 3D standard (par exemple du type OBJ, wrl, stl, ...).

Pour obtenir un rendu optimal au niveau de la texture de la reconstruction 3D, en particulier un rendu des couleurs de l'objet ou de la scène, des moyens d'éclairage peuvent être installés dans la cabine afin de fournir en toute circonstance un éclairage uniforme sur l'objet dont la surface volumique ou l'enveloppe est à acquérir.

La présente invention a également pour objet une installation 6 pour la mise en oeuvre du procédé décrit précédemment et comprenant une structure support 3 sur laquelle sont montées une pluralité de capteurs 4 et de repères visuels 5, et qui délimite un espace d'acquisition 2, notamment adapté pour accueillir un sujet humain en position debout ou assise, et éventuellement toute position autorisant le relevé de mesures particulières.

Comme le montrent les figures 1 à 4, les différents capteurs 4 sont tous situés en dehors de l'espace d'acquisition 2 et sont arrangés pour couvrir ensemble la totalité de cet espace, avec un recouvrement partiel périphérique des champs d'acquisition 7 entre capteurs 4 voisins, et sont aptes à projeter un motif de points dans un spectre non visible, préférentiellement infrarouge, et à fournir les coordonnées spatiales (x, y, z) dans son référentiel spatial propre, par exemple avec comme origine le centre optique du capteur 4 concerné, de chaque point d'une surface tridimensionnelle 1 visible dans son champ d'acquisition 7.

L'installation 6 comprend en outre au moins une unité 9 de traitement, gérant le fonctionnement de l'installation 6, apte à transformer les coordonnées spatiales des points des surfaces 1 acquises par chaque capteur 4 en des coordonnées dans un référentiel spatial unique commun, associé à l'installation 6, préférentiellement à l'espace d'acquisition 2, ce par l'intermédiaire de matrices de conversion ou de transformation, paramétrés dans une phase de calibration et d'étalonnage de l'installation 6, et apte à fusionner les représentations des surfaces 1 acquises par les différents capteurs 4, en une unique représentation tridimensionnelle 1 globale par assemblage ordonnée, avec fusion naturelle au niveau de leurs zones périphériques chevauchantes 8.

L'origine du référentiel spatial unique peut par exemple correspondre au centre géométrique de l'espace d'acquisition 2.

Conformément à l'invention, l'installation est caractérisée en ce que les repères visuels 5, actifs, sont positionnés aux limites de l'espace d'acquisition 2, ce de manière répartie, chaque capteur 4 observant au moins quatre repères visuels 5 actifs non coplanaires qui sont situés dans son champ d'acquisition 7, en ce que les repères visuels 5 se présentent sous la forme de voyants lumineux, par exemple de diodes électroluminescentes, dont les axes d'émission sont avantageusement orientés perpendiculairement par rapport à l'axe optique 4' de l'un au moins des capteurs 4 aptes à les englober dans leurs champs d'acquisition 7, et en ce que l'unité de traitement 9 comprend ou est associée à un moyen de commande individuelle de chaque voyant formant repère 5, apte à appliquer une séquence d'allumage et d'extinction spécifique et propre à chaque repère 5 et à moduler séparément et indépendamment en intensité chaque voyant formant repère 5, jusqu'à aboutir à une représentation ponctuelle du repère 5 considéré dans l'image ou la représentation de ce repère 5 acquise par les capteurs 4 dans les champs d'acquisition 7 desquels il est situé.

En accord avec un mode de réalisation préféré de l'invention, illustré sur les figures annexées, l'espace d'acquisition 2 consiste en un volume parallélépipédique rectangle avec une forme et des dimensions adaptées pour loger un sujet humain en position debout verticale et/ou autre, cet espace 2 étant délimité par des montants 10 portant chacun certains repères visuels 5, par exemple quatre, six ou huit montants verticaux 10 faisant partie de la structure support 3 et arrangés au sol au niveau des angles et côtés d'un carré ou d'un rectangle, les capteurs 4 étant préférentiellement installés en dehors et au-delà desdits montants 10 formant arêtes angulaires pour l'espace d'acquisition 2, en étant par exemple positionnés au moins au niveau des arêtes angulaires d'un parallélépipède de plus grande dimension et entourant le parallélépipède formé par les montants 10, et orientés de telle manière qu'au moins trois montants 10 soient partiellement situés dans le champ d'acquisition 7 de chaque capteur 4, avec une partie au moins de leur hauteur.

En vue d'aboutir à une répartition des capteurs 4 autorisant une acquisition optimale du sujet, de l'objet ou de la scène situé dans l'espace d'acquisition 2, il est avantageusement prévu que les capteurs 4, et préférentiellement également les repères lumineux 5, soient arrangés de manière superposée en plusieurs niveaux ou étages, en étant répartis par colonnes ou rampes 11 s'étendant selon la hauteur de l'espace d'acquisition 2, les capteurs 4 du niveau ou étage supérieur étant avantageusement orientés de manière inclinée vers le bas (notamment pour permettre une bonne acquisition du dessus des épaules dans le cas d'un sujet humain) et les capteurs 4 du niveau ou étage inférieur étant avantageusement orientés de manière inclinée vers le haut.

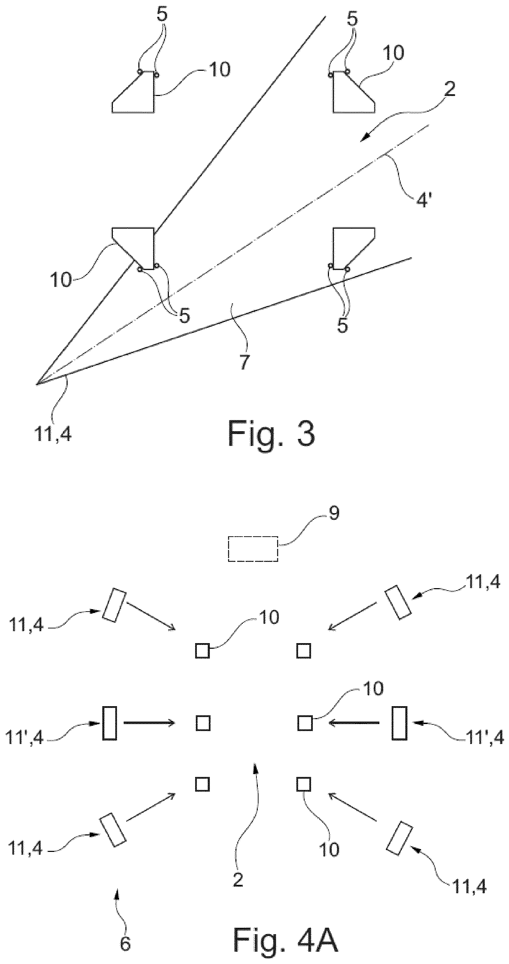

Selon une autre caractéristique de l'invention, ressortant notamment des figures 2 et 3 des dessins annexés, les capteurs 4, en fonction de la forme et des dimensions de leur champ d'acquisition 7 respectif, sont, d'une part, positionnés et orientés à chaque étage ou niveau de telle manière qu'ils englobent chacun dans leur champ d'acquisition respectif 7 le montant 10 le plus éloigné d'eux et que leur axe optique 4' passe sensiblement par le milieu de l'un des côtés latéraux de l'espace d'acquisition 2 contigus à l'arête angulaire formée par le montant 10 de plus proche d'eux, et, d'autre part, arrangés, le long de la hauteur de l'espace d'acquisition 2 et à chaque niveau ou étage, de telle manière qu'il existe une superposition partielle périphérique de leur champ d'acquisition 7 avec le champ 7 de chacun des capteurs 4 qui lui sont immédiatement voisins ou adjacents.

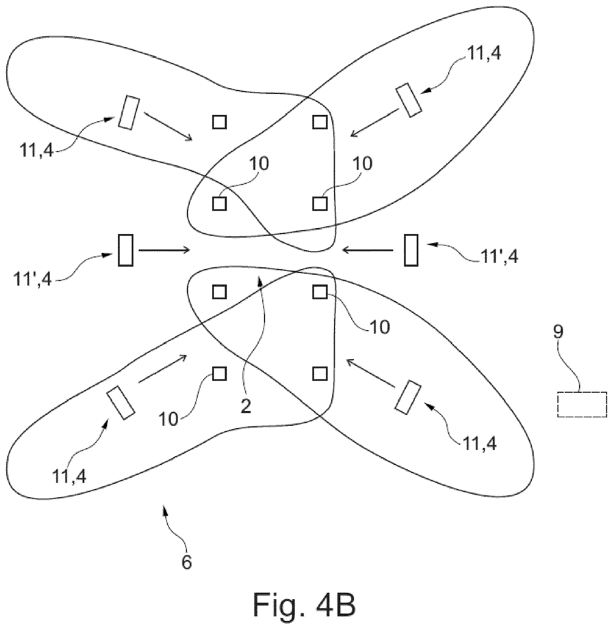

Conformément à une variante de réalisation de l'invention, et comme le montrent schématiquement les figures 1B et 4, l'installation 6 peut comprendre deux colonnes 11' de capteurs 4 superposés supplémentaires, disposées chacune en face de l'un des deux grands côtés opposés de l'espace d'acquisition 2, lorsque ce dernier a une forme parallélépipédique à section rectangulaire.

Comme le montrent en outre, par comparaison, les figures 3, 4A et 4B, l'installation 6 peut comporter, en fonction de la portée et des formes des champs d'acquisition 7 des capteurs 4, ainsi que de leur arrangement par rapport à l'espace d'acquisition 2, quatre, six ou huit montants 10 pourvus de repères 5 répartis sur leur hauteurs (de manière régulière, ou par grappes en fonction de la localisation des capteurs 4). La figure 4B montre également les différents triplets de montants 10 dont les repères 5 sont pris en compte par chacune des colonnes 11 de capteurs 4 situées aux angles de l'espace d'acquisition 2.

Les repères visuels actifs 5 peuvent être avantageusement montés dans un boîtier, derrière un cache ou dans l'intérieur (éventuellement creux) des montants 10 et projeter leur rayonnement visible à travers un orifice de faible taille, assimilé sensiblement à un pixel sur les images acquises par les capteurs 4 (émission lumineuse ponctuelle à l'emplacement précis déterminé dans le repère de l'installation).

Les repères 5 sont arrangés sur les différentes faces des montants 10 visibles pour l'un ou plusieurs des capteurs 4.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention comme définit dans les revendications.

## Revendications

1. Procédé d'acquisition automatique, de traitement et de restitution numérique de plusieurs surfaces tridimensionnelles délimitant par coopération une enveloppe d'un corps physique ou une scène, en particulier l'enveloppe d'un corps humain entier, dans lequel le sujet dont l'enveloppe est à relever est placé ou se place dans un espace d'acquisition situé dans une structure support pour des moyens capteurs et des éléments de repères visuels faisant partie d'une installation d'acquisition, de traitement, de restitution et de stockage de représentations d'enveloppes ou de scènes tridimensionnelles,
ladite installation comprenant, d'une part, une pluralité de capteurs situés en dehors de l'espace d'acquisition et arrangés pour couvrir ensemble la totalité de cet espace avec recouvrement partiel périphérique de leurs champs d'acquisition individuels, et, d'autre part, des repères visuels actifs positionnés aux limites de l'espace d'acquisition, de manière répartie, et parmi lesquels des repères sont situés dans le champ d'acquisition de chacun des capteurs, chaque capteur étant apte à projeter un motif de points dans le spectre non visible et à fournir les coordonnées (x, y, z) dans son repère ou référentiel spatial de chaque point d'une surface tridimensionnelle visible dans son champ d'acquisition,
ce procédé consistant à réaliser une phase d'étalonnage et de calibration de l'installation (6), pendant laquelle des matrices de transformation ou de conversion de coordonnées entre chacun des repères ou référentiels spécifiques aux différents capteurs (4) et un repère ou un référentiel spatial unique associé à l'installation (6) sont calculées, ce pour chaque nouvelle acquisition de représentation de surfaces tridimensionnelles (1) par les différents capteurs (4), à convertir les coordonnées des voxels desdites représentations numériques partielles dans le référentiel spatial associée à l'installation (6) et à constituer la représentation tridimensionnelle (1') complète de l'enveloppe ou de la scène par assemblage ordonné des différentes représentations partielles avec fusion naturelle desdites surfaces au niveau de leurs zones périphériques mutuellement superposées ou chevauchantes (8),
procédé **caractérisé en ce que** la phase d'étalonnage et de calibration est réalisée automatiquement, sur la base de l'acquisition des coordonnées d'au moins quatre repères visuels (5) actifs non coplanaires par chaque capteur (4), et **en ce qu'**il consiste, durant cette phase, à identifier les repères visuels actifs (5), sous forme de points lumineux, par un codage temporel, en appliquant une séance d'allumage et d'extinction déterminée unique pour chaque repère (5) et à moduler individuellement en intensité chaque repère visuel (5), sous forme de point lumineux, jusqu'à ce que l'image de ce repère (5) acquise par le capteur (4) concerné corresponde à un unique pixel.

2. Procédé selon la revendication 1, **caractérisé en ce qu'**il consiste à positionner spatialement les repères visuels (5) par rapport aux différents capteurs (4) de telle manière que parmi les au moins quatre repères (5) non coplanaires vus par chaque capteur (4), au moins un est situé dans la région centrale du champ d'acquisition (7) du capteur (4) concerné et au moins un, préférentiellement plusieurs, dans une zone périphérique dudit champ (7).

3. Procédé selon l'une quelconque des revendications 1 et 2, **caractérisé en ce que** deux capteurs (4) voisins, dont les champs d'acquisition (7) se chevauchent partiellement en périphérie, partagent au moins un repère visuel commun (5) situé dans ladite région de chevauchement mutuel, lesdits repères visuels actifs (5) se présentant préférentiellement chacun sous la forme d'une diode électroluminescente, éventuellement disposée derrière une surface perforée et dont l'axe d'émission est avantageusement disposé perpendiculairement à l'axe optique du ou des capteurs (4) dont le(s) champ(s) d'acquisition (7) l'englobe(nt).

4. Procédé selon l'une quelconque des revendications 1 à 3, **caractérisé en ce qu'**il consiste à réaliser virtuellement au moins une projection planaire des points de la représentation numérique tridimensionnelle globale (1') sur au moins un plan, à en extraire une ou plusieurs ligne(s) de contour et à effectuer des mesures et/ou des localisations de zones ou de points remarquables, sur cette ou ces lignes.

5. Installation pour la mise en oeuvre du procédé selon l'une quelconque des revendications 1 à 4, comprenant une structure support (3), une pluralité de capteurs (4) et de repères visuels (5) montés sur ladite structure support (3), cette dernière délimitant un espace d'acquisition (2), notamment adapté pour accueillir un sujet humain en position debout ou assise,
les différents capteurs (4) étant tous situés en dehors de l'espace d'acquisition (2) et sont arrangés pour couvrir ensemble la totalité de cet espace, avec un recouvrement partiel périphérique des champs d'acquisition (7) entre capteurs (4) voisins, en étant apte à projeter un motif de points dans un spectre non visible, préférentiellement infrarouge, et à fournir les coordonnées spatiales (x, y, z) dans son référentiel spatial propre, par exemple avec comme origine le centre optique du capteur (4) concerné, de chaque point d'une surface tridimensionnelle (1) visible dans son champ d'acquisition (7), l'installation (6) comprenant en outre au moins une unité (9) de traitement, gérant le fonctionnement de l'installation (6), apte à transformer les coordonnées spatiales des points des surfaces (1) acquises par chaque capteur (4) en des coordonnées dans un référentiel spatial unique commun, associé à l'installation (6), préférentiellement à l'espace d'acquisition (2), ce par l'intermédiaire de matrices de conversion ou de transformation, paramétrés dans une phase de calibration et d'étalonnage de l'installation (6), et apte à fusionner les représentations des surfaces (1) acquises par les différents capteurs (4), en une unique représentation tridimensionnelle (1) globale par assemblage ordonnée, avec fusion naturelle au niveau de leurs zones périphériques chevauchantes (8), l'installation **caractérisée en ce que** les repères visuels (5), actifs, sont positionnés aux limites de l'espace d'acquisition (2), ce de manière répartie, chaque capteur (4) comportant au moins quatre repères visuels (5) actifs non coplanaires situés dans son champ d'acquisition (7),
**en ce que** les repères visuels (5) se présentent sous la forme de voyants lumineux, par exemple de diodes électroluminescentes, dont les axes d'émission sont avantageusement orientés perpendiculairement par rapport à l'axe optique (4') de l'un au moins des capteurs (4) aptes à les englober dans leurs champs d'acquisition (7),
et **en ce que** l'unité de traitement (9) comprend ou est associée à un moyen de commande individuelle de chaque voyant formant repère (5), ledit moyen de commande formant partie de l'installation et étant configuré à appliquer une séquence d'allumage et d'extinction spécifique et propre à chaque repère (5) et à moduler séparément et indépendamment en intensité chaque voyant formant repère (5), jusqu'à aboutir à une représentation ponctuelle du repère (5) considéré dans l'image ou la représentation de ce repère (5) acquise par les capteurs (4) dans les champs d'acquisition (7) desquels il est situé.

6. Installation selon la revendication 5, **caractérisée en ce que** l'espace d'acquisition (2) consiste en un volume parallélépipédique rectangle avec une forme et des dimensions adaptées pour loger un sujet humain en position debout verticale et/ou autre, cet espace (2) étant délimité par des montants (10) portant chacun certains repères visuels (5), par exemple quatre, six ou huit montants verticaux (10) faisant partie de la structure support (3) et arrangés au sol au niveau des angles et côtés d'un carré ou d'un rectangle, les capteurs (4) étant préférentiellement installés en dehors et au-delà desdits montants (10) formant arêtes angulaires pour l'espace d'acquisition (2), en étant par exemple positionnés au moins au niveau des arêtes angulaires d'un parallélépipède de plus grande dimension et entourant le parallélépipède formé par les montants (10), et orientés de telle manière qu'au moins trois montants (10) soient partiellement situés dans le champ d'acquisition (7) de chaque capteur (4), avec une partie au moins de leur hauteur.

7. Installation selon la revendication 5 ou 6, **caractérisée en ce que** les capteurs (4), et préférentiellement également les repères visuels (5), sont arrangés de manière superposée en plusieurs niveaux ou étages, en étant répartis par colonnes (11) s'étendant selon la hauteur de l'espace d'acquisition (2), les capteurs (4) du niveau ou étage supérieur étant avantageusement orientés de manière inclinée vers le bas et les capteurs (4) du niveau ou étage inférieur étant avantageusement orientés de manière inclinée vers le haut.

8. Installation selon la revendication 6 ou 7, **caractérisée en ce que** les capteurs (4), en fonction de la forme et des dimensions de leur champ d'acquisition (7) respectif, sont, d'une part, positionnés et orientés à chaque étage ou niveau de telle manière qu'ils englobent chacun dans leur champ d'acquisition respectif (7) le montant (10) le plus éloigné d'eux et que leur axe optique (4') passe sensiblement par le milieu de l'un des côtés latéraux de l'espace d'acquisition (2) contigus à l'arête angulaire formée par le montant (10) de plus proche d'eux, et, d'autre part, arrangés, le long de la hauteur de l'espace d'acquisition (2) et à chaque niveau ou étage, de telle manière qu'il existe une superposition partielle périphérique de leur champ d'acquisition (7) avec le champ (7) de chacun des capteurs (4) qui lui sont immédiatement voisins ou adjacents.

9. Installation selon l'une quelconque des revendications 6 à 8, **caractérisée en ce qu'**elle comprend deux colonnes (11') de capteurs (4) superposés supplémentaires, disposées chacune en face de l'un des deux grands côtés opposés de l'espace d'acquisition (2), lorsque ce dernier a une forme parallélépipédique à section rectangulaire.

## Patentansprüche

1. Verfahren zur automatischen Erfassung, Verarbeitung und digitalen Wiedergabe mehrerer dreidimensionaler Oberflächen, die zusammenwirkend eine Hülle eines physischen Körpers oder eine Szene begrenzen, insbesondere die Hülle eines ganzen menschlichen Körpers, in dem das Motiv, dessen Hülle zu erfassen ist, in einen Erfassungsbereich gebracht wird oder sich begibt, der sich in einer Tragekonstruktion für Aufnahmemittel und visuelle Markierungsmittel befindet, die Teil einer Anlage zur Erfassung, Verarbeitung, Wiedergabe und Speicherung von Abbildungen dreidimensionaler Hüllen oder Szenen sind,
wobei die genannte Anlage einerseits mehrere Aufnahmemittel umfasst, die sich außerhalb des Erfassungsbereiches befinden und dafür angeordnet sind, zusammen diesen gesamten Bereich mit partiellen peripheren Überlappungen ihrer jeweiligen Erfassungsfelder abzudecken, und andererseits aktive visuelle Markierungen, die an den Grenzen des Erfassungsbereiches verteilt angeordnet sind und unter denen sich Markierungen im Erfassungsfeld jedes der Aufnahmemittel befinden, wobei jedes Aufnahmemittel in der Lage ist, ein Punktmotiv im nicht sichtbaren Spektralbereich zu projizieren und Koordinaten (x, y, z) in seinem räumlichen Bezugssystem von jedem Punkt einer dreidimensionalen Oberfläche auszugeben, die in seinem Erfassungsfeld sichtbar ist,
wobei dieses Verfahren darin besteht, einen Schritt zur Eichung und Kalibrierung der Anlage (6) auszuführen, in dem Koordinatentransformations- oder -konversionsmatrizen zwischen jedem der räumlichen Bezugssysteme, die den verschiedenen Aufnahmemitteln (4) eigen sind, und einem einzigen räumlichen Bezugssystem der Anlage (6) berechnet werden, dies für jede neue Erfassung einer Darstellung dreidimensionaler Oberflächen (1) durch die verschiedenen Aufnahmemittel (4), um die Koordinaten der Voxel der genannten partiellen digitalen Darstellungen in das räumliche Bezugssystem der Anlage (6) zu konvertieren, und die vollständige dreidimensionale Abbildung (1') der Hülle oder der Szene durch geordnetes Zusammenfügen der verschiedenen partiellen Darstellungen mit natürlicher Verschmelzung der genannten Oberflächen in ihren peripheren wechselseitigen Überlappungsbereichen (8) zu erstellen, Verfahren, **dadurch gekennzeichnet, dass** der Eich- und Kalibrierschritt automatisch aufgrund der Erfassung der Koordinaten mindestens vierer nicht koplanarer, aktiver visuellen Markierungen (5) je Aufnahmemittel (4) erfolgt, und dadurch, dass es darin besteht, in diesem Schritt die aktiven visuellen Markierungen (5) in Form von Lichtpunkten durch eine zeitliche Kodierung zu identifizieren, indem eine für jede Markierung (5) festgelegte, einzigartige An- und Ausschaltfolge angewendet wird und die Intensität jeder visuellen Markierung (5) in Form eines Lichtpunktes individuell moduliert wird, bis das vom betroffenen Aufnahmemittel (4) erfasste Bild dieser Markierung einem einzigen Pixel entspricht.

2. Verfahren nach Patentanspruch 1, **dadurch gekennzeichnet, dass** es darin besteht, die visuellen Markierungen (5) relativ zu den verschiedenen Aufnahmemitteln (4) derart anzuordnen, dass unter den mindestens vier nicht-koplanaren Markierungen (5), die von jedem Aufnahmemittel (4) gesehen werden, sich mindestens eine im Mittelbereich des Erfassungsfeldes (7) des betroffenen Aufnahmemittels (4) befindet, und mindestens eine, vorzugsweise mehrere, in einem peripheren Bereich des genannten Feldes (7).

3. Verfahren nach irgendeinem der Patentansprüche 1 und 2, **dadurch gekennzeichnet, dass** zwei benachbarte Aufnahmemittel (4), deren Erfassungsfelder sich partiell peripher überlappen, mindestens eine visuelle Markierung (5) gemeinsam haben, die sich im genannten Bereich wechselseitiger Überlappung befindet, wobei die genannten aktiven visuellen Markierungen (5) vorzugsweise jeweils die Form einer Leuchtdiode aufweisen, die gegebenenfalls hinter einer perforierten Fläche angeordnet ist und deren Abstrahlungsachse vorteilhafterweise senkrecht zur optischen Achse des oder der Aufnahmemittel (4) angeordnet ist, in dessen/deren Erfassungsfeld(ern) (7) sie sich befindet.

4. Verfahren nach irgendeinem der Patentansprüche 1 bis 3, **dadurch gekennzeichnet, dass** es darin besteht, mindestens eine planare Projektion der Punkte der globalen dreidimensionalen digitalen Darstellung (1') in mindestens eine Ebene virtuell auszuführen, daraus eine oder mehrere Konturlinie(n) zu extrahieren und Messungen und/oder Ortsbestimmungen besonderer Bereiche oder Punkte auf dieser/n Linie(n) vorzuriehmen.

5. Anlage zur Ausführung des Verfahrens nach irgendeinem der Patentansprüche 1 bis 4, eine Tragekonstruktion (3), mehrere Aufnahmemittel (4) und visuelle Markierungen (5) an der genannten Tragekonstruktion (3) angebracht umfassend, wobei diese letztere einen Erfassungsbereich (2) umgrenzt, der insbesondere dafür eingerichtet ist, einen Menschen in aufrechter oder sitzender Stellung aufzunehmen,
wobei sich alle verschiedenen Aufnahmemittel (4) außerhalb des Erfassungsbereichs (2) befinden und derart angeordnet sind, dass sie gemeinsam diesen gesamten Bereich abdecken, unter partieller peripherer Überlappung der Erfassungsfelder (7) benachbarter Aufnahmemittel (4), und dabei in der Lage sind, ein Punktmuster in einem nicht-sichtbaren Spektrum, vorzugsweise Infrarot, zu projizieren und in ihrem eigenen räumlichen Bezugssystem, beispielsweise mit dem optischen Zentrum des betroffenen Aufnahmemittels (4) als Ursprung, die räumlichen Koordinaten (x, y, z) jedes Punktes einer dreidimensionalen Oberfläche (1), die in seinem Erfassungsfeld (7) sichtbar ist, auszugeben,
wobei die Anlage (6) außerdem mindestens eine Verarbeitungseinheit (9) umfasst, die die Arbeit der Anlage (6) leitet und in der Lage ist, die räumlichen Koordinaten der Punkte der Oberflächen (1), die von jedem Aufnahmemittel (4) erfasst wurden, in Koordinaten in einem einzigen gemeinsamen räumlichen Bezugssystem zu transformieren, und zwar mit Hilfe von Konvertierungs- oder Transformationsmatrizen, die in einem Schritt zur Eichung und Kalibrierung der Anlage (6) bestimmt wurden, und in der Lage, die von den verschiedenen Aufnahmemitteln (4) erfassten Darstellungen der Oberflächen (1) zu einer einzigen globalen dreidimensionalen Darstellung (1) durch geordnetes Zusammenfügen zu verschmelzen, unter natürlicher Verschmelzung in ihren überlappenden peripheren Bereichen (8),
Anlage, **dadurch gekennzeichnet, dass** die aktiven visuellen Markierungen (5) an den Grenzen des Erfassungsbereichs (2) verteilt angeordnet sind und jedes Aufnahmemittel (4) mindestens vier nicht koplanare, aktive, visuelle Markierungen (5) in seinem Erfassungsfeld (7) aufweist,
dadurch, dass die visuellen Markierungen (5) die Form von Anzeigelampen, beispielsweise Leuchtdioden, aufweisen, deren Abstrahlachsen vorteilhafterweise rechtwinklig zur optischen Achse (4') mindestens eines der Aufnahmemittel (4) orientiert sind, die in der Lage sind, sie in ihr Erfassungsfeld (7) einzubeziehen,
und dadurch, dass die Verarbeitungseinheit (9) ein Mittel zur individuellen Betätigung jeder Anzeigelampe, die eine Markierung (5) bildet, umfasst oder damit verbunden ist, wobei das genannte Betätigungsmittel Teil der Anlage ist und dafür ausgelegt ist, eine spezifische und eigene An- und Ausschaltfolge an jede Markierung (5) anzulegen und die Intensität jeder eine Markierung (5) bildenden Anzeigelampe getrennt und unabhängig zu modulieren, bis eine punktförmigen Darstellung der betrachteten Markierung (5) im von den Aufnahmemitteln (4), in deren Erfassungsfeld (7) sie sich befindet, erfassten Bild oder Darstellung dieser Markierung (5) erzielt ist.

6. Anlage nach Patentanspruch 5, **dadurch gekennzeichnet, dass** der Erfassungsbereich (2) aus einem quaderförmigen Raum besteht mit einer Form und Abmessungen, die geeignet sind, einen Menschen in einer aufrechten und/oder anderen Stellung aufzunehmen, wobei dieser Bereich (2) durch Pfosten (10) umgrenzt wird, die jeweils bestimmte visuellen Markierungen (5) tragen, wobei beispielsweise vier, sechs oder acht vertikale Pfosten (10) Teil der Tragekonstruktion (3) sind und am Boden im Bereich der Winkel und Seiten eines Quadrates oder Rechteckes angeordnet sind, wobei die Aufnahmemittel (4) vorzugsweise außerhalb und jenseits der genannten Pfosten (10) angebracht sind, die die Eckkanten des Erfassungsbereiches (2) bilden, indem sie beispielsweise mindestens an den Eckkanten eines Quaders größerer Abmessungen angeordnet sind und den von den Pfosten (10) gebildeten Quader umgeben und derart orientiert sind, dass sich mindestens drei Pfosten (10) teilweise, über mindestens einen Teil ihrer Höhe, im Erfassungsfeld (7) jedes Aufnahmemittels (4) befinden.

7. Anlage nach Patentanspruch 5 oder 6, **dadurch gekennzeichnet, dass** die Aufnahmemittel (4) und vorzugsweise ebenfalls die visuellen Markierungen (5) übereinander in mehreren Niveaus oder Stufen angeordnet sind, in Form von Spalten (11) verteilt, die sich über die Höhe des Erfassungsbereiches (2) erstrecken,
wobei die Aufnahmemittel (4) des obersten Niveaus oder Stufe vorteilhafterweise nach unten geneigt sind und die Aufnahmemittel (4) des unteren Niveaus oder Stufe vorteilhafterweise nach oben geneigt sind.

8. Anlage nach Patentanspruch 6 oder 7, **dadurch gekennzeichnet, dass** die Aufnahmemittel (4) in Abhängigkeit von Form und Abmessungen ihres jeweiligen Erfassungsfeldes (7) einerseits auf jedem Niveau oder jeder Stufe derart angeordnet und orientiert sind, dass jedes von ihnen in seinem jeweiligen Erfassungsfeld (7) den von ihnen am weitesten entfernten Pfosten (10) enthalten und dass ihre optische Achse (4') im Wesentlichen durch die Mitte einer der Seiten des Erfassungsbereichs (2) anschließend an die Eckkante, die vom ihnen nächstgelegenen Pfosten (10) gebildet wird, verläuft und andererseits über die Höhe des Erfassungsbereichs (2) und in jedem Niveau oder jeder Stufe derart angeordnet sind, dass eine partielle periphere Überschneidung ihres Erfassungsfeldes (7) mit dem Feld (7) jedes der Aufnahmemittel (4) besteht, die ihm unmittelbar benachbart oder anschließend sind.

9. Anlage nach irgendeinem der Patentansprüche 6 bis 8, **dadurch gekennzeichnet, dass** sie zwei zusätzliche Säulen (11') von Aufnahmemitteln (4) umfasst, die sich jeweils einer der beiden großen, gegenüberliegenden Seiten des Erfassungsbereichs (2) gegenüber befinden, wenn dieser eine Quaderform mit rechteckigem Querschnitt hat.

## Claims

1. Method for automatic acquisition, processing and digital reproduction of a plurality of three-dimensional surfaces cooperating to delimit an envelope of a physical body or a scene, in particular the envelope of a complete human body, in which method the subject whose envelope is to be scanned is placed or places themselves in an acquisition space situated in a support structure for sensing means and visual marker elements forming part of an installation for acquisition, processing, reproduction and storage of three-dimensional representations of envelopes or scenes,
said installation comprising, on the one hand, a plurality of sensors situated outside the acquisition space and arranged to cover together the whole of that space with partial peripheral overlapping of their individual acquisition fields and, on the other hand, active visual markers positioned in a distributed manner at the limits of the acquisition space and among which there are markers situated in the acquisition field of each of the sensors, each sensor being able to project a pattern of points in the invisible spectrum and to supply the coordinates (x, y, z) in its system of axes or spatial frame of reference of each point of a three-dimensional surface visible in its acquisition field,
this method consisting in executing a phase of calibration of the installation (6) during which matrices for transformation or conversion of coordinates between each of the systems of axes or frames of reference specific to the various sensors (4) and a single system of axes or spatial frame of reference associated with the installation (6) are calculated for each new acquisition for representation of three-dimensional surfaces (1) by the various sensors (4) and in converting the coordinates of the voxels of said partial digital representations in the spatial frame of reference associated with the installation (6) and in constituting the complete three-dimensional representation (1') of the envelope or of the scene by ordered assembly of the various partial representations with natural merging of said surfaces at the level of their mutually superposed or overlapping peripheral zones (8),
method **characterized in that** the calibration phase is executed automatically on the basis of the acquisition of the coordinates of at least four non-coplanar active visual markers (5) by each sensor (4) and **in that**, during that phase, it consists in identifying the active visual markers (5) in the form of spots of light by temporal coding by applying a unique particular sequence of lighting and extinction for each marker (5) and individually modulating the intensity of each visual marker (5) in the form of spots of light until the image of that marker (5) acquired by the sensor (4) concerned corresponds to a single pixel.

2. Method according to Claim 1, **characterized in that** it consists in spatially positioning the visual markers (5) relative to the various sensors (4) so that of the at least four non-coplanar markers (5) seen by each sensor (4) at least one is situated in the central region of the acquisition field (7) of the sensor (4) concerned and at least one of them and preferably several of them is or are situated in a peripheral zone of said field (7).

3. Method according to either one of Claims 1 and 2, **characterized in that** two adjacent sensors (4) the acquisition fields (7) of which partially overlap at their periphery share at least one common visual marker (5) situated in said mutual overlapping region, said active visual markers (5) preferably each taking the form of a light-emitting diode, possibly disposed behind a perforated surface and the emission axis of which is advantageously disposed perpendicularly to the optical axis of the sensor or sensors (4) the acquisition field(s) (7) of which surround(s) it.

4. Method according to any one of Claims 1 to 3, **characterized in that** it consists in executing virtually at least one planar projection of the points of the overall three-dimensional digital representation (1') onto at least one plane, extracting one or more contour lines therefrom and executing measurements and/or localizations of remarkable zones or points on that line or those lines.

5. Installation for implementing the method according to any one of Claims 1 to 4, comprising a support structure (3), a plurality of sensors (4) and visual markers (5) mounted on said support structure (3), the latter delimiting an acquisition space (2) notably adapted to receive a standing or seated human subject,
the various sensors (4) being all situated outside the acquisition space (2) and arranged to cover the whole of that space with partial peripheral overlapping of the acquisition fields (7) between adjacent sensors (4), adapted to project a pattern of points in a non-visible, preferably infrared, spectrum and to supply the spatial coordinates (x, y, z) in its own spatial frame of reference, for example with as origin the optical centre of the sensor (4) concerned, of each point of a three-dimensional surface (1) visible in its acquisition field (7),
the installation (6) further comprising at least one processing unit (9) managing the operation of the installation (6) and able to transform the spatial coordinates of the points of the surfaces (1) acquired by each sensor (4) into coordinates in a common single spatial frame of reference associated with the installation (6), preferably with the acquisition space (2), by means of conversion or transformation matrices with parameters set in a phase of calibration of the installation (6) and able to merge the representations of the surfaces (1) acquired by the various sensors (4) into a single overall three-dimensional representation (1) by ordered assembly with natural merging at the level of their overlapping peripheral zones (8),
the installation **characterized in that** the active visual markers (5) are positioned in a distributed manner at the limits of the acquisition space (2), each sensor (4) including at least four non-coplanar active visual markers (5) situated in its acquisition field (7),
**in that** the visual markers (5) take the form of indicator lights, for example light-emitting diodes, the emission axes of which are advantageously oriented perpendicularly to the optical axis (4') of at least one of the sensors (4) with their acquisition fields (7) adapted to surround them,
and **in that** the processing unit (9) comprises or is associated with individual control means for each lamp forming a marker (5),
said control means forming part of the installation and being configured to apply a sequence of lighting and extinction specific to each marker (5) and to modulate separately and independently the intensity of each lamp forming a marker (5) until arriving at a point representation of the marker (5) concerned in the image or the representation of that marker (5) acquired by the sensors (4) in the acquisition fields (7) of which it is situated.

6. Installation according to Claim 5, **characterized in that** the acquisition space (2) consists in a rectangular parallelepipedal volume with a shape and dimensions adapted to accommodate a human subject in a vertical standing and/or other position, that space (2) being delimited by uprights (10) each carrying certain visual markers (5), for example four, six or eight vertical uprights (10) forming part of the support structure (3) and arranged on the floor at the level of the corners and sides of a square or a rectangle, the sensors (4) preferably being installed outside and beyond said uprights (10) forming corner edges for the acquisition space (2) and being for example positioned at least at the level of the corner edges of a larger parallelepiped surrounding the parallelepiped formed by the uprights (10) and oriented so that at least a part of the height of at least three uprights (10) is partially situated in the acquisition field (7) of each sensor (4).

7. Installation according to Claim 5 or 6, **characterized in that** the sensors (4), and preferably also the visual markers (5), are arranged in a superposed manner on several levels or tiers, being distributed in columns (11) extending along the height of the acquisition space (2), the sensors (4) of the upper level or tier advantageously being oriented in a downwardly inclined manner and the sensors (4) of the lower level or tier advantageously being oriented in an upwardly inclined manner.

8. Installation according to Claim 6 or 7, **characterized in that** the sensors (4), depending on the shape and the dimensions of their respective acquisition fields (7), are, on the one hand, positioned and oriented on each tier or level so that they each surround with their respective acquisition field (7) the upright (10) farthest from them and their optical axis (4') passes substantially through the middle of one of the lateral sides of the acquisition space (2) contiguous with the corner edge formed by the upright (10) closest to them and, on the other hand, arranged along the height of the acquisition space (2) and at each level or tier so that there exists a partial peripheral superposition of their acquisition field (7) with the field (7) of each of the immediately neighbouring or adjacent sensors (4).

9. Installation according to any one of Claims 6 to 8, **characterized in that** it comprises two columns (11') of superposed additional sensors (4) each disposed facing one of the two opposite large sides of the acquisition space (2) when the latter has a parallelepipedal shape of rectangular section.
